(19) Europäisches Patentamt / European Patent Office / Office européen des brevets

(11) **EP 3 207 372 B1**

(12) **EUROPÄISCHE PATENTSCHRIFT**

(45) Veröffentlichungstag und Bekanntmachung des Hinweises auf die Patenterteilung:
**23.01.2019 Patentblatt 2019/04**

(21) Anmeldenummer: **15780895.7**

(22) Anmeldetag: **15.10.2015**

(51) Int Cl.:
**G01N 33/543** (2006.01)    **G01N 33/92** (2006.01)

(86) Internationale Anmeldenummer:
**PCT/EP2015/073902**

(87) Internationale Veröffentlichungsnummer:
**WO 2016/059164 (21.04.2016 Gazette 2016/16)**

(54) **BESTIMMUNG VON BINDUNGSKONSTANTEN MITTELS GLEICHGEWICHTSVERLAGERUNG**

DETERMINATION OF BINDING CONSTANTS BY EQUILIBRIUM SHIFT

DÉTERMINATION DE CONSTANTES DE LIAISON PAR TRANSFERT D'ÉQUILIBRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.10.2014 DE 102014115088**

(43) Veröffentlichungstag der Anmeldung:
**23.08.2017 Patentblatt 2017/34**

(73) Patentinhaber: **3B Pharmaceuticals GmbH 12489 Berlin (DE)**

(72) Erfinder: **BORISS, Hinnerk 04155 Leipzig (DE)**

(74) Vertreter: **Patentanwälte Olbricht Buchhold Keulertz Partnerschaft mbB Bettinastraße 53-55 60325 Frankfurt am Main (DE)**

(56) Entgegenhaltungen:
**WO-A1-2005/017528    WO-A1-2011/134860**

- JOACHIM SCHUHMACHER ET AL: "High-throughput determination of the free fraction of drugs strongly bound to plasma proteins", JOURNAL OF PHARMACEUTICAL SCIENCES, Bd. 93, Nr. 4, 1. April 2004 (2004-04-01), Seiten 816-830, XP055236254, WASHINGTON, US ISSN: 0022-3549, DOI: 10.1002/jps.10588
- R. LONGHI ET AL: "Brain Tissue Binding of Drugs: Evaluation and Validation of Solid Supported Porcine Brain Membrane Vesicles (TRANSIL) as a Novel High-Throughput Method", DRUG METABOLISM AND DISPOSITION, Bd. 39, Nr. 2, 11. November 2010 (2010-11-11), Seiten 312-321, XP055236256, US ISSN: 0090-9556, DOI: 10.1124/dmd.110.036095
- SCHUHMACHER J ET AL: "Determination of the free fraction and relative free fraction of drugs strongly bound to plasma proteins", JOURNAL OF PHARMACEUTICAL SCIENCES, AMERICAN PHARMACEUTICAL ASSOCIATION, WASHINGTON, US, Bd. 89, Nr. 8, 1. August 2000 (2000-08-01), Seiten 1008-1021, XP002301862, ISSN: 0022-3549, DOI: 10.1002/1520-6017(200008)89:8<1008::AID-JPS5>3.0.CO;2-B
- KIM YUMEE ET AL: "A phospholipase A-2 kinetic and binding assay using phospholipid-coated hydrophobic beads", ANALYTICAL BIOCHEMISTRY, ACADEMIC PRESS INC, NEW YORK, Bd. 250, Nr. 1, 1. Januar 1997 (1997-01-01), Seiten 109-116, XP002546382, ISSN: 0003-2697, DOI: 10.1006/ABIO.1997.2200
- LOIDL-STAHLHOFEN A ET AL: "Multilamellar liposomes and solid-supported lipid membranes (TRANSIL): screening of lipid-water partitioning toward a high-throughput scale", PHARMACEUTICAL RESEARCH, SPRINGER NEW YORK LLC, US, Bd. 18, Nr. 12, 1. Dezember 2001 (2001-12-01), Seiten 1782-1788, XP002527409, ISSN: 0724-8741, DOI: 10.1023/A:1013343117979

- **VICTOR TUAN GIAM CHUANG ET AL: "Updates on Contemporary Protein Binding Techniques", DRUG METABOLISM AND PHARMACOKINETICS, Bd. 24, Nr. 4, 12. Juni 2009 (2009-06-12), Seiten 358-364, XP055236261, JP ISSN: 1347-4367, DOI: 10.2133/dmpk.24.358**
- **HARTMANN T ET AL: "Lipophilicity - beyond octanol/water: a short comparison of modern technologies", DRUG DISCOVERY TODAY: TECHNOLOGIES, ELSEVIER, AMSTERDAM, NL, Bd. 1, Nr. 4, 1. Dezember 2004 (2004-12-01), Seiten 431-439, XP004767947, ISSN: 1740-6749, DOI: 10.1016/J.DDTEC.2004.10.006**
- **HONG WAN ET AL: "High-Throughput Screening of Drug-Brain Tissue Binding and in Silico Prediction for Assessment of Central Nervous System Drug Delivery", JOURNAL OF MEDICINAL CHEMISTRY, Bd. 50, Nr. 19, 29. März 2007 (2007-03-29) , Seiten 4606-4615, XP055236267, US ISSN: 0022-2623, DOI: 10.1021/jm070375w**

**Beschreibung**

Die Erfindung betrifft ein Verfahren zur Bestimmung von Bindungskonstanten zwischen einer Substanz oder einem Substanzgemisch und einem Target gemäß Anspruch 1. Die Bestimmung von Wechselwirkungen und Bindungskonstanten zwischen einer Substanz oder einem Substanzgemisch und einem Target spielt eine große Rolle in der pharmazeutischen Industrie, insbesondere in der pharmakologischen Forschung und Entwicklung. Beide Anwendungsgebiete befassen sich mit Wechselwirkungen zwischen Substanz oder Substanzgemischen und Lebewesen. Dabei ist es essentiell, die Bindungseigenschaften einzelner Substanzen mit Bindungsparametern und Bindungskonstanten zu quantifizieren.

Ein Verfahren zur Bestimmung von Bindungskonstanten ist aus der DE 10 2010 018 965 A1 bekannt. Bei diesem Verfahren werden z.B. auf Festkörpern immobilisierte Proteine mit einer wässrigen Lösung der zu untersuchenden Substanz in Kontakt gebracht und für eine zur Bindung der gelösten Substanz an die Proteine ausreichende Zeit inkubiert. Danach werden die festkörperunterstützten Proteine aus der Lösung abgetrennt (z.B. durch Sedimentation). Die Konzentration der zu untersuchenden Substanz wird in der verbleibenden Lösung (Überstand) oder gegebenenfalls auch in den separierten festkörperunterstützten Proteinen durch geeignete Messverfahren bestimmt, und zwar für mehrere verschiedene Mengenverhältnisse von festkörperunterstützter Proteine zu untersuchender Substanz. Dazu werden gleiche Mengen der auf Bindung zu untersuchenden Substanz in einheitlichen Lösungsvolumina mit unterschiedlichen Mengen an festkörperunterstützten Proteinen inkubiert. Das Gesamtvolumen der flüssigen Phase der zu inkubierenden Probe, das aus dem Volumen der Pufferlösung besteht, in der die zu untersuchende Substanz oder das zu untersuchende Substanzgemisch gelöst ist, wurde dabei stets konstant gehalten. Nachteilig an diesem Verfahren ist, dass Substanzen, die sehr hohe Affinitäten zu Kunststoff oder Glas aufweisen, unspezifisch an den Probenbehältnissen kleben und somit verloren gehen, oder aufgrund geringer Löslichkeit ausfallen.

Weiterhin wird in EP 1 658 499 A1 ein Verfahren zur Bestimmung der Bindungskonstanten bzw. der freien Fraktion von Substanzen in verdünntem Plasma und Serum beschrieben, in dem die Bindung einer Substanz an immobilisierte Membranen (Target 1) und verdünntes Plasma (Target 2) bestimmt wird. Nachteilig an diesem Verfahren ist, dass keine Wechselwirkungen zwischen Substanzen und Targets bestimmt werden können, die eine extrem hohe oder eine sehr geringe Affinität an Membranen aufweisen. Letzteres ist zum Beispiel der Fall bei Peptiden, die z.B. mit Fettsäuren als Lipidanker konjugiert sind, während ersteres häufig bei Lipiden ohne solche Anker der Fall ist.

WO 2005/017528 beschreibt die Bestimmung freier Fraktionen von pharmakologisch aktiven Substanzen in wässrigen Lösungen und Serum. Der vorliegenden Erfindung lag daher die Aufgabe zugrunde, ein Verfahren zur Bestimmung von Bindungskonstanten zwischen einer Substanz oder einem Substanzgemisch und einem Target bereitzustellen, das die mit den bekannten Verfahren verbundenen Nachteile überwindet. Insbesondere sollen auch Bindungskonstanten zwischen Substanzen und Targets bestimmbar sein, die bei Anwendung bisheriger Verfahren eine extrem hohe oder eine sehr geringe Affinität an Membranen oder eine extrem hohe Affinität zum Probenbehältnis aufweisen. Nach einem weiteren Aspekt sollte zudem ein Verfahren bereitgestellt werden, bei dem die Bindungskonstanten zwischen der Substanz und dem immobilisierten Target sowie der Substanz und dem löslichen Target gleichzeitig bestimmbar sind.

Diese Aufgabe wird gelöst durch das Verfahren mit den Merkmalen des Anspruchs 1. Ausgestaltungen sind Gegenstand der Ansprüche 2 bis 13. Die Erfindung betrifft ein Verfahren zur Bestimmung von Bindungskonstanten einer Substanz oder eines Substanzgemisches in Bezug auf ein lösliches Target und ein unlösliches Target, umfassend die Schritte

- Inkubieren einer ersten Probe der Substanz oder des Substanzgemisches mit einem auf einem festen, vorzugsweise partikulären, Träger immobilisierten Target in einem ersten Probenbehältnis, welches eine Pufferlösung und ein gelöstes Target enthält,
- Inkubieren einer zweiten Probe der Substanz oder des Substanzgemisches mit dem auf einem festen, vorzugsweise partikulären, Träger immobilisierten Target in einem zweiten Probenbehältnis, welches Pufferlösung und gelöstes Target enthält,
- Inkubieren einer dritten Probe der Substanz oder des Substanzgemisches mit dem auf einem festen, vorzugsweise partikulären, Träger immobilisierten Target in einem dritten Probenbehältnis, welches Pufferlösung und gelöstes Target enthält,
- Inkubieren einer vierten Probe der Substanz oder des Substanzgemisches mit dem auf einem festen, vorzugsweise partikulären, Träger immobilisierten Target in einem vierten Probenbehältnis, welches Pufferlösung und gelöstes Target enthält,

dabei ist erfindungsgemäß vorgesehen, dass die erste und die zweite Probe der Substanz mit unterschiedlichen Stoffmengen des immobilisierten Targets und der gleichen Stoffmenge des gelösten Targets inkubiert werden, die dritte und die vierte Probe der Substanz mit den Stoffmengen des immobilisierten Targets wie die erste und die zweite Probe der Substanz und mit einer gleichen Stoffmenge des löslichen Targets inkubiert werden, wobei sich die Stoffmenge des löslichen Targets in den Probenbehältnissen drei und vier von der Stoffmenge des löslichen Targets in den Probenbehältnissen eins und zwei unterscheidet, und die Probenbehältnisse bei der Inkubation das gleiche Volumen an flüssiger Phase, bestehend aus Pufferlösung, gelöstem Target und Substanzprobe, enthalten und das erfindungsgemäße Verfahren weiterhin die folgenden Schritte umfasst:

- Abtrennen des festen, vorzugsweise partikulären, Trägers aus den jeweiligen Inkubationsansätzen,

- Messung der Konzentration der nicht an das immobilisierte Target gebundenen Substanz oder des nicht an das immobilisierte Target gebundenen Substanzgemisches (APA-Konzentration) im Überstand des jeweiligen Inkubationsansatzes,

- Bestimmung einer Bindungskonstante der Substanz oder des Substanzgemisches zum immobilisierten Target und einer Bindungskonstante der Substanz oder des Substanzgemisches zum gelösten Target mit Hilfe der gemessenen APA-Konzentrationen.

Die Erfindung betrifft insbesondere ein Verfahren zur Bestimmung von Bindungskonstanten zwischen einer einzigen Substanz und einem Target. Im Sinne der vorliegenden Erfindung können die Bindungskonstanten jedoch in Alternative dazu auch für ein Substanzgemisch zu einem Target bestimmt werden. Unter einem Substanzgemisch ist hier insbesondere ein solches Gemisch zu verstehen, das mindestens zwei Substanzen enthält, die gegebenenfalls an ein Target binden. Für jede Substanz in einem solchen Substanzgemisch kann mit dem erfindungsgemäßen Verfahren die Bindungskonstante in Bezug auf das Target bestimmt werden. Dies kommt insbesondere beim Multiplexing zur Anwendung. Wenn nachfolgend nicht explizit differenziert wird, dann ist bei Verwendung des Begriffs Substanz auch das Substanzgemisch mit umfasst.

Grundlegendes Funktionsprinzip des erfindungsgemäßen Verfahrens ist die Bestimmung der Bindungskonstanten der Substanz an die Targets durch Verlagerung des Bindungsgleichgewichts. Dabei werden die Konzentrationen beider Targets, nämlich des immobilisierten und des gelösten Targets, variiert und durch die Verschiebung der Bindungsgleichgewichte in den einzelnen Ansätzen die Affinitäten an die Targets bestimmt.

Das erfindungsgemäße Verfahren dient somit zur Bestimmung von Bindungskonstanten zwischen einer Substanz oder einem Substanzgemisch und einem Target. Allerdings wird bei dem erfindungsgemäßen Verfahren im Gegensatz zum Stand der Technik nicht nur die Konzentration des immobilisierten Targets variiert, sondern auch eine zweite Phase des selben oder unterschiedlichen Target in Lösung eingeführt, deren Konzentration ebenfalls variiert wird. Vorteil des erfindungsgemäßen Verfahrens ist somit, dass auch Wechselwirkungen zwischen einer Substanz oder einem Substanzgemisch und zwei unterschiedlichen Targets bestimmt werden können. Weiterhin erhöht die Einführung des zweiten gelösten Targets die Löslichkeit der Substanzen oder Substanzgemische. Zur Bestimmung der Bindungskonstanten werden mindestens vier Proben benötigt: zwei für unterschiedliche Stoffmengen des immobilisierten Targets kombiniert mit zwei unterschiedlichen Stoffmengen des löslichen Targets. Dies ergibt die Mindestmenge an gemessenen Konzentrationswerte der Substanz im Überstand (frei oder an lösliches Target gebunden), die benötigt wird, um die erfindungsgemäß die Bindungskonstanten der Substanz an das immobilisierte und gelöste Target zu bestimmen. Durch die Tatsache, dass das erfindungsgemäße Verfahren neben dem immobilisierten Target zudem ein lösliches Target aufweist und nicht nur die Konzentration des immobilisierten Targets sondern auch des löslichen Targets variiert wird, ist es durch das erfindungsgemäße Verfahren möglich, gleichzeitig die Bindungskonstanten zwischen der Substanz und dem immobilisierten Target und die Bindungskonstanten zwischen der Substanz und dem löslichen Target zu bestimmen.

Nach einem wesentlichen Aspekt der vorliegenden Erfindung wird zum Zweck der Bestimmung der Bindungskonstanten das Bindungsgleichgewicht verlagert. Dabei werden die Konzentrationen des immobilisierten und des gelösten Targets variiert. Die erste und die zweite Probe der Substanz werden mit unterschiedlichen Stoffmengen des immobilisierten Targets inkubiert. Hierdurch unterscheidet sich die Stoffmenge des immobilisierten Targets in Inkubationsansatz eins von der Stoffmenge des immobilisierten Targets in Inkubationsansatz zwei. Weiterhin werden die dritte und die vierte Probe der Substanz mit einer unterschiedlichen Stoffmenge des löslichen Targets inkubiert. Hierdurch unterscheidet sich die Stoffmenge des löslichen Targets in Inkubationsansatz drei und vier von der Stoffmenge des löslichen Targets in Inkubationsansatz eins und zwei. Der jeweilige Unterschied der Stoffmenge zum Erreichen der genannten Varianz der Konzentration der Targets kann jeweils, unabhängig voneinander, in einem Bereich von Faktor 1,5 bis 100, insbesondere 2 bis 10, liegen.

Die dritte und die vierte Probe der Substanz werden mit den gleichen Stoffmengen des immobilisierten Targets wie die erste und die zweite Probe der Substanz inkubiert. Im Ergebnis wird somit in den Inkubationsansätzen drei und vier die Stoffmenge des immobilisierten Targets wie in den Inkubationsansätzen eins und zwei variiert.

Daraus folgt mit anderen Worten, dass die Inkubationsansätze eins und drei die gleiche Stoffmenge des immobilisierten Targets aufweisen. Die Inkubationsansätze zwei und vier weisen ebenfalls die gleiche Stoffmenge des immobilisierten Targets auf, die sich aber von der Stoffmenge des immobilisierten Targets der Inkubationsansätze eins und drei unterscheidet. Inkubationsansätze eins und zwei enthalten die gleiche Stoffmenge des gelösten Targets und auch die Inkubationsansätze drei und vier enthalten die gleiche Stoffmenge des gelösten Targets, wobei sich letztere Stoffmenge von der Stoffmenge der Inkubationsansätze eins und zwei jedoch unterscheidet.

Bei der Messung der APA-Konzentration wird die Konzentration der nicht an das immobilisierte Target gebundenen Substanz oder des nicht an das immobilisierte Target gebundenen Substanzgemisches im Überstand des jeweiligen Inkubationsansatzes bestimmt. Es handelt sich bei der APA-Konzentration um die gesamte Konzentration der nicht an das immobilisierte Target gebundenen Substanz oder des nicht an das immobilisierte Target gebundenen Substanzgemisches im Überstand des jeweiligen Inkubationsansatzes, also umfassend freie Substanz oder freies Substanzgemisch und an lösliches Target gebundene Substanz oder an lösliches Target gebundenes Substanzgemisch.

Man erkennt, dass nach dem erfindungsgemäßen Verfahren mindestens vier APA-Konzentrationen bestimmt werden, da mindestens vier Probenbehältnisse mit entsprechenden Inkubationsansätzen eingesetzt werden.

Hierin beschrieben ist, dass zusätzlich zur ersten, zweiten, dritten und vierten Probe der Substanz mindestens eine weitere Probe der Substanz oder des Substanzgemisches, vorzugsweise zwischen 1 und 21 weitere Proben, mit dem auf einem festen, vorzugsweise partikulären Träger immobilisierten Target in mindestens einem weiteren Pufferlösung und gelöstes Target enthaltenden Probenbehältnis, vorzugsweise in 1 - 21 weiteren Pufferlösung enthaltenden Probenbehältnissen, inkubiert wird, wobei die weiteren Proben mit unterschiedlichen Stoffmengen des immobilisierten Targets inkubiert werden und die weiteren Proben mit unterschiedlichen Stoffmengen des gelösten Targets inkubiert werden und mindestens eine der weiteren Proben in zwei Probenbehältnissen mit der gleichen Konzentration von immobilisiertem Target und gleichzeitig unterschiedlicher Konzentrationen von gelöstem Target inkubiert wird und alle weiteren Probenbehältnisse bei der Inkubation die gleiche Menge Pufferlösung enthalten wie das erste, zweite, dritte und vierte Probenbehältnis. Vorteil dieser Erhöhung der Probenanzahl ist, dass die Fehlerquote sinkt je mehr Konzentrationswerte zur Bestimmung herangezogen werden können.

Zur Abschätzung der Bindungskonstanten wird gegebenenfalls in jeweils 2 bis 5 Reaktionsansätzen die immobilisierte Targetkonzentration variiert und eine gleichbleibende Menge Substanz hinzugegeben. Weiterhin wird zu diesen Reaktionsansätzen ein gleichbleibendes Volumen des gelösten Targets (Plasma, Albumin, etc.) hinzugegeben. Dabei ist zu beachten, dass das gelöste Target in dem Volumen durch Verdünnung mit Puffer unterschiedliche Konzentrationen aufweisen kann. In 2 bis 5 parallelen Ansätzen werden bis zu 5 verschiedene Konzentrationen gelöster Targets eingesetzt, so dass 4 bis 25 Reaktionsansätze mit unterschiedlichen Konzentrationen an immobilisiertem Target und gelösten Target entstehen.

Wie hierin beschrieben, werden zusätzlich zur ersten, zweiten, dritten und vierten Substanzprobe mindestens fünf weitere Proben der Substanz oder des Substanzgemisches verwendet (also 9 Proben, das entspricht 3 Konzentrationsstufen des immobilisierten Targets in Kombination mit 3 Konzentrationsstufen des löslichen Targets). Vorzugsweise werden 25 Proben verwendet. Dabei werden 5 unterschiedliche Stoffmengen des immobilisierten Targets mit 5 unterschiedlichen Stoffmengen des löslichen Targets kombiniert, so dass jede einzelne Stoffmenge des immobilisierten Targets mit jeder Stoffmenge des löslichen Targets inkubiert wird und so sich 25 verschiedene Bindungsgleichgewichte einstellen. Stets enthalten die Probenbehältnisse bei der Inkubation jedoch die gleiche Menge an Pufferlösung wie das erste, zweite, dritte und vierte Probenbehältnis. Zur Abschätzung der Bindungskostanten wird in 25, Reaktionsansätzen die Targetkonzentration variiert und eine gleichbleibende Substanzmenge hinzugegeben. Dabei kommen mindestens zwei verschiedene Konzentrationen beider, also gelöster und immobilisierter, Targets zum Einsatz.

Eine weitere Ausführung sieht vor, dass die Konzentration der Substanz oder des Substanzgemisches (APA) im Überstand des jeweiligen Inkubationsansatzes relativ zu einer Referenzprobe bestimmt wird. Bei einer solchen Referenzprobe handelt es sich insbesondere um eine Probe, die nur Pufferlösung, lösliches Target und die zu untersuchende Substanz oder das zu untersuchende Substanzmischung enthält, nicht aber das immobilisierte Target.

Nach einer Ausgestaltung des erfindungsgemäßen Verfahrens sind das gelöste und das immobilisierte Target identisch. Nach einer weiteren Abwandlung sind das gelöste und das immobilisierte Target unterschiedlich.

Bei einem erfindungsgemäßen Target handelt es sich bevorzugt um eine Zielverbindung oder einen Zielrezeptor. Die Targets können somit auch als Rezeptoren bezeichnet werden. An das Target können beispielsweise Wirk- oder Schadstoffe binden. Als Targets sind insbesondere biologische Targets wie Proteine, vorzugsweise Enzyme, Antikörper oder Gemische, wie Plasma, geeignet. Bei den Targets kann es sich insbesondere um nicht-membrangebundene Proteine oder für die Bindung verantwortliche extrazelluläre Protein-Domänen wie *Epidermal Growth Factor Receptor,* (EGFR) und *Tumor Necrosis Factor Receptor* (TNFR) oder auch um Plasmaproteine, wie *Human serum albumin* (HSA) oder *alpha-1-acid glycoprotein* (AGP), Proteingemische, wie Plasma oder Serum, oder Gewebehomogenate handeln. Bei den Targets kann es sich um natürlich vorkommende oder um künstlich hergestellte Targets handeln. Man erkennt, dass auch das Target ein Gemisch aus verschiedenen Zielverbindungen oder Zielrezeptoren sein kann. Allen immobilisierten Targets ist dabei gemein, dass sie auf einem Festkörper immobilisiert sind.

Die zu testende Substanz oder das Substanzgemisch kann auch als Ligand bezeichnet werden. Dabei kann es sich bei der zu untersuchenden Substanz um Peptide, Nukleinsäuren, Ribonukleinsäuren, Lipide und andere Biomoleküle handeln. Weiterhin kann es sich bei den zu testenden Substanzen um chemische Substanzen, wie beispielsweise Arzneistoffe oder Toxine, handeln. Bei der Interaktion zwischen Target und Substanz oder Substanzgemisch kann es sich um spezifische oder unspezifische Bindungen handeln. Die Bindungen sind im Allgemeinen nicht kovalent. Dabei konkurrieren die Testsubstanzen in der Bindung mit den Targets. Der Einsatz von immobilisierten Targets, wie Proteinen, wie beispielsweise Albumin oder Antikörpern, ermöglicht gegenüber der Verwendung von Membranen, die Bestimmung der Wechselwirkungen zwischen Substanzen und Targets, die nur eine sehr geringe oder eine extrem hohe Affinität an Membranen aufweisen. Solche Substanzen sind zum Beispiel Peptide.

Im erfindungsgemäßen Verfahren handelt es sich bei den zu bestimmenden Bindungskonstanten um Affinitäts- bzw. Dissoziationskonstanten.

Die Bestimmung der Affinitäts- bzw. Dissoziationskonstanten erfolgt im erfindungsgemäßen Verfahren mit Hilfe der Gleichung I:

$$APA = \frac{c_0 \cdot K_D^H \cdot ([P] + K_D^P)}{[\text{immoT}] \cdot K_D^P + K_D^H \cdot ([P] + K_D^P)} \quad \text{(I)},$$

wobei

APA die Konzentration der Substanz bezeichnet, die nicht an das immobilisierte Target gebunden ist, d.h. freie Substanz und an lösliches Target gebundene Substanz,

$K_D^H$ die Dissoziationskonstante von dem immobilisierten Target,

$K_D^P$ die Dissoziationskonstante von dem gelösten Target,

$c_0$ die gesamte konstant zugegebene Substanzkonzentration,

[immoT] die Konzentration des immobilisierten Targets und

[P] die Konzentration des gelösten Targets bezeichnet.

Danach wird somit die Dissoziationskonstante der Substanz in Bezug auf das immobilisierte Target sowie die Dissoziationskonstante der Substanz in Bezug auf das gelöste Target bestimmt. Es ist bekannt, dass sich die jeweilige Assoziationskonstante aus der Dissoziationskonstante rechnerisch ergibt und umgekehrt.

Zur Anwendung der Gleichung müssen die Wechselwirkungen zwischen Substanz und den Targets einer Bindungskinetik erster Ordnung oder näherungsweise erster Ordnung folgen.

Die Bestimmung der Dissoziationskonstanten erfolgt somit mit Hilfe eines Verfahrens nach einer der oben genannten Ausführungsformen, wobei vorzugsweise weiterhin folgende Schritte umfasst sind:

- Einsetzen einer Matrix von Dissoziationskonstanten für $K_D^H$ und $K_D^P$ in die Gleichung I,
- Berechnung der jeweiligen zu erwartenden APA Konzentrationen für die Matrix von Dissoziationskonstanten,
- Vergleich der berechneten APA Konzentrationen mit der gemessenen APA Konzentration,
- Auswahl des Wertepaars $K_D^H$ und $K_D^P$, das zur kleinsten Abweichung zwischen berechneter APA Konzentration und gemessener APA Konzentration führt als bestimmte Dissoziationskonstanten der zu untersuchenden Substanz in Bezug auf das immobilisierte bzw. das gelöste Target.

Bei einer bevorzugten Ausführung erfolgt dabei die Auswahl des Wertepaares $K_D^H$ und $K_D^P$, das zur kleinsten Abweichung zwischen berechneter APA Konzentration und gemessener APA Konzentration führt, durch ein numerisches Optimierungsverfahren.

Nach einer Weiterentwicklung des erfindungsgemäßen Verfahrens erfolgt die Bestimmung der Bindungskonstanten mit der genannten Gleichung $APA = \frac{c_0 \cdot K_D^H \cdot ([P] + K_D^P)}{[\text{immoT}] \cdot K_D^P + K_D^H \cdot ([P] + K_D^P)}$, wobei zunächst mehrere Bindungskonstantenwerte für $K_D^H$ und $K_D^P$ in die Gleichung eingesetzt werden und die jeweiligen zu erwartenden APA Konzentrationen für diese Werte berechnet werden. Danach werden die berechneten APA Konzentrationen mit der gemessenen APA Konzentration verglichen. In einem weiteren Schritt wird die kleinste Abweichung zwischen der berechneten APA Konzentration und der gemessenen APA Konzentration mit Hilfe eines numerischen Optimierungsverfahrens, vorzugsweise des "kleinste Quadrate" Optimierungsverfahrens und der Formel $r = (APA_{gemessen} - APA_{berechnet})^2$, bestimmt. Aus diesen Abweichungen wird der geringste Wert für die Abweichung ausgewählt. Danach erfolgt die Auswahl des zugehörigen Wertepaars für die Bindungskonstanten $K_D^H$ und $K_D^P$ dem dieser Wert der geringsten Abweichung zuzuordnen ist. Dabei handelt es sich dann um die Bindungskonstanten der zu untersuchenden Substanz.

So kann nach einer bevorzugten Ausführung eine Matrix von Dissoziationskonstanten $K_D^H$ und $K_D^P$ errichtet werden. Die Matrix wird mit den Residuen gefüllt, die sich aus den gemessenen APA-Werten und aus den $K_D^H$ und $K_D^P$ Werten der jeweiligen Zeile und Spalte der Matrix berechneten APA-Werten nach der folgenden Formel ergeben:

$$r = \left(APA_{gemessen} - APA_{berechnet}\right)^2$$

Die Kombination aus $K_D^H$ und $K_D^P$ Werten, die zu der kleinsten Abweichung führt, stellt die optimale Kombination aus $K_D^H$ und $K_D^P$ Werten dar, die die Messwerte am besten erklärt. Die so bestimmten $K_D^H$ und $K_D^P$ Werte sind die erfindungsgemäß erhaltenen Bindungskonstanten.

In einer Ausführungsform des Verfahrens ist ein Träger für das immobilisierte Target vorgesehen, der in wässriger Lösung unlöslich ist. Dadurch lässt sich der Träger leicht vom Inkubationsansatz abtrennen und ist gegebenenfalls für eine nochmalige Durchführung des Verfahrens wiedereinsetzbar.

In einer besonders bevorzugten Ausführungsform besteht der Träger aus organischem oder anorganischem Polymer, wobei insbesondere Agarose besonders bevorzugt wird. Der Vorteil von Agarose besteht in der geringen unspezifischen Bindung von zu testenden Substanzen, wie insbesondere von Peptiden.

Nach einer weiteren Ausführungsform des erfindungsgemäßen Verfahrens ist vorgesehen, dass der Träger partikulär vorliegt, wobei die Partikel zumindest teilweise mikro- oder nanoskalig sind. Dabei können die Partikel magnetische oder nicht-magnetische Nanopartikel, Silikapartikel oder Sepharose-Mikrokugeln sein.

In einer besonders bevorzugten Ausgestaltung des Verfahrens ist der Träger ein partikulärer Agarose-Träger. Der Vorteil eines solchen Trägers besteht weiterhin darin, dass dieser kommerziell bezogen werden kann.

Besonders bevorzugt ist eine Ausführungsvariante des erfindungsgemäßen Verfahrens bei dem das immobilisierte Target Albumin ist. Albumin eignet sich besonders gut als immobilisiertes Target, da es nach Standardverfahren immobilisiert werden kann.

In einer weiteren vorteilhaften Ausgestaltung des Verfahrens ist das lösliche Target Plasma oder Serum von humanem oder tierischem Ursprung. Der Vorteil von Plasma oder Serum besteht in der besonderen physiologischen Relevanz der Messergebnisse.

Eine weitere Ausführung des erfindungsgemäßen Verfahrens sieht vor, dass das Abtrennen des Trägers durch Filtration, Zentrifugation oder Dekantieren der Pufferlösung erfolgt. Durch solche Verfahren lässt sich der Träger ohne erhöhten Zeit- und Kostenaufwand vom Inkubationsansatz abtrennen.

In einer bevorzugten Ausführungsform liegt der Träger partikulär vor und die Partikel weisen magnetische Eigenschaften auf. Dabei ist nach einer erfindungsgemäßen Ausgestaltung vorgesehen, dass das Abtrennen des Trägers durch Separierung des Trägers durch Anlegen eines magnetischen Feldes erfolgt. Der Einsatz magnetischer Partikel und der Magnetseparation hat gegenüber anderen Techniken erhebliche

Vorteile. Die magnetischen Partikel können direkt aus dem Inkubationsansatz selektiv isoliert und aufgereinigt werden. Im Vergleich zu konventionellen Methoden zur Separation ist die Magnetseparation einfach und schnell. Zudem lassen sich Vorgänge wie Pufferwechsel oder Waschschritte einfach realisieren.

Besonders bevorzugt erfolgt die Messung der Konzentration der Substanz oder des Substanzgemisches durch massenspektrometrische, fluoreszenzspektroskopische, radioaktive oder chromatographische Verfahren oder eine Kombination dieser Verfahren. Diese Verfahren sind vielfältig abwandelbar und lassen sich je nach Testsubstanz entsprechend anpassen und modifizieren. Auf diese Weise lässt sich jede Konzentration der Substanz möglichst genau nachweisen.

Nach einer weiteren Ausgestaltung des erfindungsgemäßen Verfahrens ist die Pufferlösung eine wässrige, salzhaltige Lösung. Solche Lösungen eignen sich besonders zur Untersuchung von Bindungskostanten, da die verwendeten Targets darin stabil sind und die Lösungen kommerziell erhältlich sind.

In einer besonders bevorzugten Ausführungsform des erfindungsgemäßen Verfahrens sind die Probenbehältnisse Kavitäten von Mikrotiterplatten oder weisen insbesondere Oberflächeneigenschaften auf, die nicht mit der Substanz oder dem Substanzgemisch interferieren.

Hierin beschrieben ist ein Kit zur Bestimmung von Bindungskonstanten zwischen einer Substanz oder einem Substanzgemisch und einem Target nach einem Verfahren gemäß einer der oben genannten Ausführungen. Dabei ist vorgesehen, dass das Kit mindestens 4 Probenbehältnisse oder mindestens 4 Kavitäten einer Mikrotiterplatte, Pufferlösung, eine Menge eines gelösten Targets und eine Menge eines auf einem festen, vorzugsweise partikulären Träger immobilisierten Targets enthält, wobei das gelöste und das immobilisierte Target identisch oder unterschiedlich sein können.

Wie hierin beschrieben ist das Verfahren zur Bestimmung von Bindungskonstanten einer Substanz oder eines Substanzgemisches in Bezug auf ein lösliches Target und ein immobilisiertes Target dahin ausgestaltet, dass insbesondere die Bestimmung einer Bindungskonstante der Substanz oder des Substanzgemisches zum immobilisierten Target und einer Bindungskonstante der Substanz oder des Substanzgemisches zum gelösten Target mit Hilfe der gemessenen APA-Konzentrationen durch Ausführung eines Computerprogramms erfolgt. Hierin beschrieben ist somit ein Computerprogramm zur Bestimmung von Bindungskonstanten einer Substanz oder eines Substanzgemisches in Bezug auf ein lösliches Target und ein immobilisiertes Target, das so ausgebildet ist, dass die Bestimmung einer Bindungskonstante der Substanz oder des Substanzgemisches zum immobilisierten Target und einer Bindungskonstante der Substanz oder des Substanzgemisches zum gelösten Target mit Hilfe der gemessenen APA-Konzentrationen ausführbar ist. Hierin beschrieben ist ein Computerprogrammprodukt, wie etwa einen Datenträger, ein Speichermedium oder ein computerlesbares Medium, mit einem solchen Computerprogramm. Das bereits genannte Kit kann erforderlichenfalls ein solches Computerprogrammprodukt zusätzlich aufweisen.

**Ausführungsbeispiel 1**:

A) Design des Experiments

Eine besonders bevorzugte Ausführung ist eine Mikrotiterplatte in der 5 Spalten mit 7 Kavitäten für das beschriebene Verfahren benutzt werden, so dass 25 Proben mit immobilisiertem Target inkubiert werden und 10 Proben als Referenzen ohne immobilisiertes Target inkubiert werden. Als immobilisiertes Target (Target 1) wurde humanes Serum-Albumin (HSA) verwendet. Das HSA wurde nach Standardverfahren auf kommerziellen Sepharose-Beads (minileak beads medium von Kem-En-Tec Nordic A/S) immobilisiert. Als lösliches Target wurde humanes Plasma verwendet. Die zu testende Substanz ist ein Peptid. Es werden für jede von 5 Plasmakonzentrationen 5 Konzentrationen von immobilisiertem HSA eingesetzt. Dazu werden die Kavitäten einer Mikrotiterplatte wie folgt gefüllt:

Spalte I der Mikrotiterplatte (unter Ausnutzung der Zeilen 1 bis 7):

|  | Kavität | | | | | | |
|---|---|---|---|---|---|---|---|
|  | I-1 | I-2 | I-3 | I-4 | I-5 | I-6 | I-7 |
|  | Ref 1 | Ref2 | V1 HSA | V2 HSA | V3 HSA | V4 HSA | V5 HSA |
| HSA-Konzentration in $\mu$M | 0 | 0 | 11 | 21 | 37 | 67 | 120 |
| Volumen der Bead Suspension in $\mu$L | 0 | 0 | 36 | 64 | 115 | 208 | 374 |
| Puffervolumen in $\mu$L | 290 | 290 | 265 | 246 | 212 | 148 | 35 |
| Volumen der Beads in $\mu$L | 0 | 0 | 11 | 20 | 37 | 66 | 119 |
| Probenvolumen in $\mu$L | 10 | 10 | 10 | 10 | 10 | 10 | 10 |
| Volumen reines Plasma in $\mu$L | 150 | 150 | 150 | 150 | 150 | 150 | 150 |

Spalte II der Mikrotiterplatte (unter Ausnutzung der Zeilen 1 bis 7):

|  | Kavität | | | | | | |
|---|---|---|---|---|---|---|---|
|  | II-1 | II-2 | II-3 | II-4 | II-5 | II-6 | II-7 |
|  | Ref 1 | Ref2 | V1 HSA | V2 HSA | V3 HSA | V4 HSA | V5 HSA |
| HSA-Konzentration in $\mu$M | 0 | 0 | 11 | 21 | 37 | 67 | 120 |
| Volumen der Bead Suspension in $\mu$L | 0 | 0 | 36 | 64 | 115 | 208 | 374 |
| Puffervolumen in $\mu$L | 290 | 290 | 265 | 246 | 212 | 148 | 35 |
| Volumen der Beads in $\mu$L | 0 | 0 | 11 | 20 | 37 | 66 | 119 |
| Probenvolumen in $\mu$L | 10 | 10 | 10 | 10 | 10 | 10 | 10 |
| Volumen 1:2 verdünntes Plasma in $\mu$L | 150 | 150 | 150 | 150 | 150 | 150 | 150 |

Spalte III der Mikrotiterplatte (unter Ausnutzung der Zeilen 1 bis 7):

| | Kavität | | | | | | |
|---|---|---|---|---|---|---|---|
| | III-1 | III-2 | III-3 | III-4 | III-5 | III-6 | III-7 |
| | Ref 1 | Ref2 | V1 HSA | V2 HSA | V3 HSA | V4 HSA | V5 HSA |
| HSA-Konzentration in µM | 0 | 0 | 11 | 21 | 37 | 67 | 120 |
| Volumen der Bead Suspension in µL | 0 | 0 | 36 | 64 | 115 | 208 | 374 |
| Puffervolumen in µL | 290 | 290 | 265 | 246 | 212 | 148 | 35 |
| Volumen der Beads in µL | 0 | 0 | 11 | 20 | 37 | 66 | 119 |
| Probenvolumen in µL | 10 | 10 | 10 | 10 | 10 | 10 | 10 |
| Volumen 1:4 verdünntes Plasma in µL | 150 | 150 | 150 | 150 | 150 | 150 | 150 |

Spalte IV der Mikrotiterplatte (unter Ausnutzung der Zeilen 1 bis 7):

| | Kavität | | | | | | |
|---|---|---|---|---|---|---|---|
| | IV-1 | IV-2 | IV-3 | IV-4 | IV-5 | IV-6 | IV-7 |
| | Ref 1 | Ref2 | V1 HSA | V2 HSA | V3 HSA | V4 HSA | V5 HSA |
| HSA-Konzentration in µM | 0 | 0 | 11 | 21 | 37 | 67 | 120 |
| Volumen der Bead Suspension in µL | 0 | 0 | 36 | 64 | 115 | 208 | 374 |
| Puffervolumen in µL | 290 | 290 | 265 | 246 | 212 | 148 | 35 |
| Volumen der Beads in µL | 0 | 0 | 11 | 20 | 37 | 66 | 119 |
| Probenvolumen in µL | 10 | 10 | 10 | 10 | 10 | 10 | 10 |
| Volumen 1:8 verdünntes Plasma in µL | 150 | 150 | 150 | 150 | 150 | 150 | 150 |

Spalte V der Mikrotiterplatte (unter Ausnutzung der Zeilen 1 bis 7):

| | Kavität | | | | | | |
|---|---|---|---|---|---|---|---|
| | V-1 | V-2 | V-3 | V-4 | V-5 | V-6 | V-7 |
| | Ref 1 | Ref2 | V1 HSA | V2 HSA | V3 HSA | V4 HSA | V5 HSA |
| HSA-Konzentration in µM | 0 | 0 | 11 | 21 | 37 | 67 | 120 |
| Volumen der Bead Suspension in µL | 0 | 0 | 36 | 64 | 115 | 208 | 374 |
| Puffervolumen in µL | 290 | 290 | 265 | 246 | 212 | 148 | 35 |
| Volumen der Beads in µL | 0 | 0 | 11 | 20 | 37 | 66 | 119 |
| Probenvolumen in µL | 10 | 10 | 10 | 10 | 10 | 10 | 10 |
| Volumen 1:16 verdünntes Plasma in µL | 150 | 150 | 150 | 150 | 150 | 150 | 150 |

Dabei wird das Plasma entsprechend der folgenden Tabelle vorverdünnt und zu den jeweiligen Kavitäten hinzugefügt:

| Plasmaverdünnung | Volumen und Konzentration des Plasmas | Hinzuzufügen zu den Kavitäten der Spalten: |
|---|---|---|
| 1:3 | 150 µL pure plasma | Spalte I |
| 1:6 | 150 µL Plasma[1] in 1: 2 Vorverdünnung | Spalte II |
| 1:12 | 150 µL Plasma[1] in 1: 4 Vorverdünnung | Spalte III |

(fortgesetzt)

| Plasmaverdünnung | Volumen und Konzentration des Plasmas | Hinzuzufügen zu den Kavitäten der Spalten: |
|---|---|---|
| 1:24 | 150 μL Plasma[1] in 1: 8 Vorverdünnung | Spalte IV |
| 1:48 | 150 μL Plasma[1] in 1: 16 Vorverdünnung | Spalte V |
| [1] Zur Berechnung der Dissoziationskonstanten wird arbiträr die Annahme gemacht, dass Vollplasma eine Konzentration von 600 μM hat. Diese Annahme kommt der Realität nahe, ist aber unkritisch, da die Berechnete Dissoziationskonstante sich auf diese Konzentration bezieht und somit korrekte Bindungswerte im Sinne von Prozent an Plasma gebundener Substanz angegeben werden auch wenn die "wahre" Konzentration anders ist. | | |

Man erkennt, dass das erfindungsgemäße Prinzip der Veränderung der Konzentrationen beider Targets, also des immobilisierten und des gelösten Targets, hier verwirklicht ist. So wird eine erste und eine zweite Probe der Substanz mit unterschiedlichen Stoffmengen des immobilisierten Targets und der gleichen Stoffmenge des gelösten Targets inkubiert (z.B. Kavität I-3 und I-7 mit einer immobilisierten HSA-Konzentration von 11 μM bzw. 120 μM, wobei beide Kavitäten 1:3 verdünntes Plasma enthalten) inkubiert. Weiterhin wird eine dritte und eine vierte Probe der Substanz mit den gleichen Stoffmengen des immobilisierten Targets wie die erste und die zweite Probe der Substanz und mit einer gleichen Stoffmenge des löslichen Targets (z.B. Kavität V-3 und V-7 mit einer immobilisierten HSA-Konzentration von 11 μM bzw. 120 μM, wobei beide Kavitäten 1:48 verdünntes Plasma enthalten) inkubiert, wobei sich die Stoffmengen des löslichen Targets in den Probenbehältnissen drei und vier von der Stoffmenge des löslichen Targets in den Probenbehältnissen eins und zwei unterscheidet.

B) Durchführung des Experiments

Zu diesen Kavitäten werden jeweils 10 μL einer 10 bis 2000 μM Stammlösung der Testsubstanzen hinzugegeben. In dem Beispiel hier wurde eine 1012,5 μM Stammlösung eines Peptids verwendet. Nach Zugabe von 10 μL zu dem oben beschriebenen Experimentellen Setup betrug die Konzentration des Peptids in allen Kavitäten 22,5 μM. Nach Mischen durch Resuspension der Beads auf denen das Target 1 (hier humanes Serum-Albumin) immobilisiert ist, wird das auf dem Trägermaterial fixierte Target abgetrennt. Dazu wird die Mikrotiterplatte zentrifugiert, so dass die Beads sedimentieren. Die Konzentration im Überstand wird durch geeignete Verfahren, wie z.B. LC/MS/MS oder Scintillationszählung, bestimmt. Die ungebundenen Fraktionen der Substanz wird in Kavitäten mit dem immobilisierten Target relativ zu den Referenzen bestimmt. Dadurch entfällt eine Kalibrierung, solange die Beziehung von Signal zu Konzentration für die Testsubstanzen und das genutzte Quantifizierungssystem linear verlaufen.

C) Auswertung des Experiments

Multipliziert man die für alle 25 Proben relativ zu den Referenzen gemessenen Konzentrationen mit der in dem Experiment eingesetzten Konzentration der Testsubstanz (hier 22.5 μM), so erhält man die APA-Konzentrationen in allen 25 Proben (dargestellt in Spalte 3). Nach der oben genannten Formel für APA lassen sich die zu erwartenden APA-Werte aus den Dissoziationskonstanten $K_D^H$ und $K_D^P$ berechnen. Um diese Dissoziationskonstanten zu bestimmen wird ein allgemein bekanntes Numerisches "kleinste Quadrate" Optimierungsverfahren angewendet. Die Ausgestaltung dieses Verfahrens kann sehr unterschiedlich sein. Für dieses Beispiel wurde eine Matrix von Dissoziationskonstanten $K_D^H$ reichend von 1.9*10⁻⁵ bis 1.1*10⁻⁷ μM und $K_D^P$ reichend von 6.0*10⁻⁴ bis 1.8*10⁻¹¹ μM errichtet. Die Matrix wurde mit den Residuen gefüllt, die sich aus den gemessenen APA-Werten und aus den $K_D^H$ und $K_D^P$ Werten der jeweiligen Zeile und Spalte der Matrix berechneten APA-Werten nach der folgenden Formel ergeben:

$$r = \left(APA_{gemessen} - APA_{berechnet}\right)^2$$

Die Kombination aus $K_D^H$ und $K_D^P$ Werten, die zu der kleinsten Abweichung führt stellt die optimale Kombination aus $K_D^H$ und $K_D^P$ Werten dar, die die Messwerte am besten erklärt. Die so bestimmten $K_D^H$ und $K_D^P$ Werte werden als Ergebnis für die Bindungskonstanten genommen. Die aus dieser Wertekombination berechneten APA-Werte sind in Spalte 4 der folgenden Tabelle dargestellt und die Residuen sind in Spalte 5 dargestellt.

| | | 1 | 2 | 3 | 4 | 5 |
|---|---|---|---|---|---|---|
| | | Konzentration des humanen Plasmas [P] in [µM] | immobilisierte HSA Konzentration [µM] | gemessene APA-Konzentration [µM] | Vorhergesagte APA-Konzentration mit optimalen Konstanten[µM] | Residuen |
| Spalte I: | | 200 | 11.4 | 20.8280 | 19.861 | 0.00277 |
| | | 200 | 20.6 | 20.9611 | 18.157 | 0.02748 |
| | | 200 | 37.0 | 17.2881 | 15.729 | 0.01665 |
| | | 200 | 66.7 | 14.4100 | 12.677 | 0.04559 |
| | | 200 | 120.0 | 10.2862 | 9.395 | 0.04305 |
| Spalte II: | | 100 | 11.4 | 20.7373 | 18.074 | 0.02555 |
| | | 100 | 20.6 | 19.6240 | 15.617 | 0.08655 |
| | | 100 | 37.0 | 15.4196 | 12.546 | 0.11166 |
| | | 100 | 66.7 | 11.7731 | 9.267 | 0.26716 |
| | | 100 | 120.0 | 6.1804 | 6.302 | 0.00492 |
| Spalte III: | | 50 | 11.4 | 19.3799 | 15.810 | 0.06874 |
| | | 50 | 20.6 | 16.5692 | 12.772 | 0.16304 |
| | | 50 | 37.0 | 12.0566 | 9.489 | 0.25492 |
| | | 50 | 66.7 | 7.0854 | 6.488 | 0.08551 |
| | | 50 | 120.0 | 3.8316 | 4.134 | 0.18473 |
| Spalte IV: | | 25 | 11.4 | 16.7864 | 13.509 | 0.10571 |
| | | 25 | 20.6 | 13.0066 | 10.237 | 0.21905 |
| | | 25 | 37.0 | 7.4661 | 7.129 | 0.02034 |
| | | 25 | 66.7 | 4.1302 | 4.609 | 0.32078 |
| | | 25 | 120.0 | 2.7004 | 2.817 | 0.11962 |
| Spalte V: | | 12.5 | 11.4 | 13.4437 | 11.643 | 0.06701 |
| | | 12.5 | 20.6 | 8.5339 | 8.400 | 0.00176 |
| | | 12.5 | 37.0 | 4.7166 | 5.595 | 0.56109 |
| | | 12.5 | 66.7 | 2.9723 | 3.495 | 1.28026 |
| | | 12.5 | 120.0 | 2.3312 | 2.085 | 1.29411 |

Die somit bestimmten Dissoziationskonstanten der Beispielsubstanz, eines Peptids, lauten für das Beispiel $K_D^P$=18,7 µM und $K_D^H$=7.3 µM. Die an humanes Vollplasma gebundene Fraktion des eingesetzten Peptids wird dann nach der allgemein bekannten Formel:

$$f_b = 1 - \frac{1}{1 + [P]/K_D^P}$$

berechnet. Wobei [P] die arbiträr gewählte Konzentration des Plasmas ist und $K_D^P$ der aus dem Optimierungsverfahren gewonnene Wert der Dissoziationskonstante des Peptids vom Plasma. In diesem Beispiel beträgt die an humanes Vollplasma (unverdünnt) gebundene Fraktion des Peptids 97%. D.h. nur 3% des im Plasma gelösten Peptids liegen frei, bzw. ungebunden, vor.

**Ausführungsbeispiel 2:**

A) Design des Experiments

Eine besonders bevorzugte Ausführung ist eine Mikrotiterplatte in der 5 Spalten mit 7 Kavitäten für das beschriebene Verfahren benutzt werden, so dass 25 Proben mit immobilisiertem Target inkubiert werden und 10 Proben als Referenzen ohne immobilisiertes Target inkubiert werden. Als immobilisiertes Target (Target 1) wurde humanes Serum-Albumin (HSA) verwendet. Das HSA wurde nach Standardverfahren auf kommerziellen Sepharose-Beads (minileak beads medium von Kem-En-Tec Nordic A/S) immobilisiert. Als lösliches Target wurde humanes Plasma verwendet. Die zu testende Substanz ist das Peptid Liraglutid. Es werden für jede von 5 Plasmakonzentrationen 5 Konzentrationen von immobilisiertem HSA eingesetzt. Dazu werden die Kavitäten einer Mikrotiterplatte wie folgt gefüllt:

Spalte I der Mikrotiterplatte (unter Ausnutzung der Zeilen 1 bis 7):

| | Kavität | | | | | | |
|---|---|---|---|---|---|---|---|
| | I-1 | I-2 | I-3 | I-4 | I-5 | I-6 | I-7 |
| | Ref 1 | Ref2 | V1 HSA | V2 HSA | V3 HSA | V4 HSA | V5 HSA |
| HSA-Konzentration in $\mu$M | 0 | 0 | 11 | 21 | 37 | 67 | 120 |
| Volumen der Bead Suspension in $\mu$L | 0 | 0 | 36 | 64 | 115 | 208 | 374 |
| Puffervolumen in $\mu$L | 290 | 290 | 265 | 246 | 212 | 148 | 35 |
| Volumen der Beads in $\mu$L | 0 | 0 | 11 | 20 | 37 | 66 | 119 |
| Probenvolumen in $\mu$L | 10 | 10 | 10 | 10 | 10 | 10 | 10 |
| Volumen reines Plasma in $\mu$L | 150 | 150 | 150 | 150 | 150 | 150 | 150 |

Spalte II der Mikrotiterplatte (unter Ausnutzung der Zeilen 1 bis 7):

| | Kavität | | | | | | |
|---|---|---|---|---|---|---|---|
| | II-1 | II-2 | II-3 | II-4 | II-5 | II-6 | II-7 |
| | Ref 1 | Ref2 | V1 HSA | V2 HSA | V3 HSA | V4 HSA | V5 HSA |
| HSA-Konzentration in $\mu$M | 0 | 0 | 11 | 21 | 37 | 67 | 120 |
| Volumen der Bead Suspension in $\mu$L | 0 | 0 | 36 | 64 | 115 | 208 | 374 |
| Puffervolumen in $\mu$L | 290 | 290 | 265 | 246 | 212 | 148 | 35 |
| Volumen der Beads in $\mu$L | 0 | 0 | 11 | 20 | 37 | 66 | 119 |
| Probenvolumen in $\mu$L | 10 | 10 | 10 | 10 | 10 | 10 | 10 |
| Volumen 1:2 verdünntes Plasma in $\mu$L | 150 | 150 | 150 | 150 | 150 | 150 | 150 |

Spalte III der Mikrotiterplatte (unter Ausnutzung der Zeilen 1 bis 7):

| | Kavität | | | | | | |
|---|---|---|---|---|---|---|---|
| | III-1 | III-2 | III-3 | III-4 | III-5 | III-6 | III-7 |
| | Ref 1 | Ref2 | V1 HSA | V2 HSA | V3 HSA | V4 HSA | V5 HSA |
| HSA-Konzentration in $\mu$M | 0 | 0 | 11 | 21 | 37 | 67 | 120 |

(fortgesetzt)

| | Kavität | | | | | | |
|---|---|---|---|---|---|---|---|
| | III-1 | III-2 | III-3 | III-4 | III-5 | III-6 | III-7 |
| | Ref 1 | Ref2 | V1 HSA | V2 HSA | V3 HSA | V4 HSA | V5 HSA |
| Volumen der Bead Suspension in $\mu$L | 0 | 0 | 36 | 64 | 115 | 208 | 374 |
| Puffervolumen in $\mu$L | 290 | 290 | 265 | 246 | 212 | 148 | 35 |
| Volumen der Beads in $\mu$L | 0 | 0 | 11 | 20 | 37 | 66 | 119 |
| Probenvolumen in $\mu$L | 10 | 10 | 10 | 10 | 10 | 10 | 10 |
| Volumen 1:4 verdünntes Plasma in $\mu$L | 150 | 150 | 150 | 150 | 150 | 150 | 150 |

Spalte IV der Mikrotiterplatte (unter Ausnutzung der Zeilen 1 bis 7):

| | Kavität | | | | | | |
|---|---|---|---|---|---|---|---|
| | IV-1 | IV-2 | IV-3 | IV-4 | IV-5 | IV-6 | IV-7 |
| | Ref 1 | Ref2 | V1 HSA | V2 HSA | V3 HSA | V4 HSA | V5 HSA |
| HSA-Konzentration in $\mu$M | 0 | 0 | 11 | 21 | 37 | 67 | 120 |
| Volumen der Bead Suspension in $\mu$L | 0 | 0 | 36 | 64 | 115 | 208 | 374 |
| Puffervolumen in $\mu$L | 290 | 290 | 265 | 246 | 212 | 148 | 35 |
| Volumen der Beads in $\mu$L | 0 | 0 | 11 | 20 | 37 | 66 | 119 |
| Probenvolumen in $\mu$L | 10 | 10 | 10 | 10 | 10 | 10 | 10 |
| Volumen 1:8 verdünntes Plasma in $\mu$L | 150 | 150 | 150 | 150 | 150 | 150 | 150 |

Spalte V der Mikrotiterplatte (unter Ausnutzung der Zeilen 1 bis 7):

| | Kavität | | | | | | |
|---|---|---|---|---|---|---|---|
| | V-1 | V-2 | V-3 | V-4 | V-5 | V-6 | V-7 |
| | Ref 1 | Ref2 | V1 HSA | V2 HSA | V3 HSA | V4 HSA | V5 HSA |
| HSA-Konzentration in $\mu$M | 0 | 0 | 11 | 21 | 37 | 67 | 120 |
| Volumen der Bead Suspension in $\mu$L | 0 | 0 | 36 | 64 | 115 | 208 | 374 |
| Puffervolumen in $\mu$L | 290 | 290 | 265 | 246 | 212 | 148 | 35 |
| Volumen der Beads in $\mu$L | 0 | 0 | 11 | 20 | 37 | 66 | 119 |
| Probenvolumen in $\mu$L | 10 | 10 | 10 | 10 | 10 | 10 | 10 |
| Volumen 1:16 verdünntes Plasma in $\mu$L | 150 | 150 | 150 | 150 | 150 | 150 | 150 |

Dabei wird das Plasma entsprechend der folgenden Tabelle vorverdünnt und zu den jeweiligen Kavitäten hinzugefügt:

| Plasmaverdünnung | Volumen und Konzentration des Plasmas | Hinzuzufügen zu den Kavitäten der Spalten: |
|---|---|---|
| 1:3 | 150 $\mu$L pure plasma | Spalte I |
| 1:6 | 150 $\mu$L Plasma in 1: 2 Vorverdünnung | Spalte II |
| 1:12 | 150 $\mu$L Plasma[1] in 1: 4 Vorverdünnung | Spalte III |
| 1:24 | 150 $\mu$L Plasma[1] in 1: 8 Vorverdünnung | Spalte IV |

(fortgesetzt)

| Plasmaverdünnung | Volumen und Konzentration des Plasmas | Hinzuzufügen zu den Kavitäten der Spalten: |
|---|---|---|
| 1:48 | 150 $\mu$L Plasma[1] in 1: 16 Vorverdünnung | Spalte V |
| [1] Zur Berechnung der Dissoziationskonstanten wird arbiträr die Annahme gemacht, dass Vollplasma eine Konzentration von 600 $\mu$M hat. Diese Annahme kommt der Realität nahe, ist aber unkritisch, da die Berechnete Dissoziationskonstante sich auf diese Konzentration bezieht und somit korrekte Bindungswerte im Sinne von Prozent an Plasma gebundener Substanz angegeben werden auch wenn die "wahre" Konzentration anders ist. | | |

Man erkennt, dass das erfindungsgemäße Prinzip der Veränderung der Konzentrationen beider Targets, also des immobilisierten und des gelösten Targets, hier verwirklicht ist. So wird eine erste und eine zweite Probe der Substanz mit unterschiedlichen Stoffmengen des immobilisierten Targets und der gleichen Stoffmenge des gelösten Targets inkubiert (z.B. Kavität V3 und V6) und eine dritte und eine vierte Probe der Substanz mit den gleichen Stoffmengen des immobilisierten Targets wie die erste und die zweite Probe der Substanz und mit einer unterschiedlichen Stoffmenge des löslichen Targets (z.B. Kavität IV3 und V3) inkubiert.

B) Durchführung des Experiments

Zu diesen Kavitäten werden jeweils 10 $\mu$L einer 0.022 $\mu$M Stammlösung der Testsubstanz Liraglutid hinzugegeben. Nach Zugabe von 10 $\mu$L zu dem oben beschriebenen Experimentellen Setup betrug die Konzentration des Peptids in allen Kavitäten 22 nM. Nach Mischen durch Resuspension der Beads auf denen das Target 1 (hier humanes Serum-Albumin) immobilisiert ist, wird das auf dem Trägermaterial fixierte Target abgetrennt. Dazu wird die Mikrotiterplatte zentrifugiert, so dass die Beads sedimentieren. Die Konzentration im Überstand wird durch geeignete Verfahren, wie z.B. LC/MS/MS oder Scintillationszählung, bestimmt. Die ungebundenen Fraktionen der Substanz wird in Kavitäten mit dem immobilisierten Target relativ zu den Referenzen bestimmt. Dadurch entfällt eine Kalibrierung, solange die Beziehung von Signal zu Konzentration für die Testsubstanzen und das genutzte Quantifizierungssystem linear verlaufen.

C) Auswertung des Experiments

Multipliziert man die für alle 25 Proben relativ zu den Referenzen gemessenen Konzentrationen mit der in dem Experiment eingesetzten Konzentration der Testsubstanz (hier 22 nM), so erhält man die APA-Konzentrationen in allen 25 Proben (dargestellt in Spalte 3). Nach der oben genannten Formel für APA lassen sich die zu erwartenden APA-Werte aus den Dissoziationskonstanten $K_D^H$ und $K_D^P$ berechnen. Um diese Dissoziationskonstanten zu bestimmen wird ein allgemein bekanntes Numerisches "kleinste Quadrate" Optimierungsverfahren angewendet. Die Ausgestaltung dieses Verfahrens kann sehr unterschiedlich sein. Für dieses Beispiel wurde eine Matrix von Dissoziationskonstanten $K_D^H$ reichend von $1.9*10^{-5}$ bis $1.1*10^{-7}$ $\mu$M und $K_D^P$ reichend von $6.0*10^{-4}$ bis $1.8*10^{-11}$ $\mu$M errichtet. Die Matrix wurde mit den Residuen gefüllt, die sich aus den gemessenen APA-Werten und aus den $K_D^H$ und $K_D^F$ Werten der jeweiligen Zeile und Spalte der Matrix berechneten APA-Werten nach der folgenden Formel ergeben:

$$r = \left(APA_{gemessen} - APA_{berechnet}\right)^2$$

Die Kombination aus $K_D^H$ und $K_D^P$ Werten, die zu der kleinsten Abweichung führt stellt die optimale Kombination aus $K_D^H$ und $K_D^P$ Werten dar, die die Messwerte am besten erklärt. Die so bestimmten $K_D^H$ und $K_D^P$ Werte werden als Ergebnis für die Bindungskonstanten genommen. Die aus dieser Wertekombination berechneten APA-Werte sind in Spalte 4 der folgenden Tabelle dargestellt und die Residuen sind in Spalte 5 dargestellt.

| | 1 | 2 | 3 | 4 | 5 |
|---|---|---|---|---|---|
| | Konzentration des humanen Plasmas [P] in [$\mu$M] | immobilisierte HSA Konzentration [$\mu$M] | gemessene APA-Konzentration [$\mu$M] | Vorhergesagte APA-Konzentration mit optimalen Konstanten[$\mu$M] | Residuen |
| Spalte I: | 200 | 11.4 | 19.9 | 22 | -2.1 |
| | 200 | 20.6 | 20.2 | 21.8 | -1.6 |
| | 200 | 37.0 | 20.5 | 21.5 | -1 |
| | 200 | 66.7 | 19.3 | 20.9 | -1.7 |
| | 200 | 120.0 | 17.2 | 20 | -2.8 |
| Spalte II: | 100 | 11.4 | 21.1 | 21.8 | -0.7 |
| | 100 | 20.6 | 22.1 | 21.4 | 0.6 |
| | 100 | 37.0 | 19.9 | 20.9 | -1 |
| | 100 | 66.7 | 18.5 | 19.9 | -1.4 |
| | 100 | 120.0 | 18.4 | 18.3 | 0 |
| Spalte III: | 50 | 11.4 | 20.8 | 21.4 | -0.6 |
| | 50 | 20.6 | 20.9 | 20.8 | 0.1 |
| | 50 | 37.0 | 19.2 | 19.8 | -0.6 |
| | 50 | 66.7 | 17.2 | 18.2 | -1.1 |
| | 50 | 120.0 | 16.3 | 15.9 | 0.3 |
| Spalte IV: | 25 | 11.4 | 21.0 | 20.8 | 0.1 |
| | 25 | 20.6 | 19.6 | 19.8 | -0.2 |
| | 25 | 37.0 | 17.8 | 18.3 | -0.5 |
| | 25 | 66.7 | 16.1 | 16 | 0.1 |
| | 25 | 120.0 | 14.7 | 13.1 | 1.6 |
| Spalte V: | 12.5 | 11.4 | 19.5 | 20 | -0.6 |
| | 12.5 | 20.6 | 18.8 | 18.6 | 0.2 |
| | 12.5 | 37.0 | 15.3 | 16.4 | -1.1 |
| | 12.5 | 66.7 | 12.8 | 13.6 | -0.8 |
| | 12.5 | 120.0 | 10.5 | 10.4 | 0.2 |

Die somit bestimmten Dissoziationskonstanten von Liraglutid, eines Peptids, lauten für das Beispiel $K_D^P$=7,53 $\mu$M und $K_D^H$=39.6 $\mu$M. Die an humanes Vollplasma gebundene Fraktion des eingesetzten Peptids wird dann nach der allgemein bekannten Formel:

$$f_b = 1 - \frac{1}{1 + [P]/K_D^P}$$

berechnet. Wobei [P] die arbiträr gewählte Konzentration des Plasmas ist und $K_D^P$ der aus dem Optimierungsverfahren gewonnene Wert der Dissoziationskonstante des Peptids vom Plasma. In diesem Beispiel beträgt die an humanes Vollplasma (unverdünnt) gebundene Fraktion des Peptids 98,8%. D.h. nur 1,2% des im Plasma gelösten Peptids liegen frei, bzw. ungebunden, vor.


**Patentansprüche**

1. Verfahren zur Bestimmung von Bindungskonstanten einer Substanz oder eines Substanzgemisches in Bezug auf ein gelöstes Target und ein immobilisiertes Target, umfassend die Schritte

   • Inkubieren einer ersten Probe der Substanz oder des Substanzgemisches mit einem auf einem festen, vorzugsweise partikulären, Träger immobilisierten Target in einem ersten Probenbehältnis, welches eine Pufferlösung und ein gelöstes Target enthält,
   • Inkubieren einer zweiten Probe der Substanz oder des Substanzgemisches mit dem auf einem festen, vorzugsweise partikulären, Träger immobilisierten Target in einem zweiten Probenbehältnis, welches Pufferlösung und gelöstes Target enthält,
   • Inkubieren einer dritten Probe der Substanz oder des Substanzgemisches mit dem auf einem festen, vorzugsweise partikulären, Träger immobilisierten Target in einem dritten Probenbehältnis, welches Pufferlösung und gelöstes Target enthält,
   • Inkubieren einer vierten Probe der Substanz oder des Substanzgemisches mit dem auf einem festen, vorzugsweise partikulären, Träger immobilisierten Target in einem vierten Probenbehältnis, welches Pufferlösung und gelöstes Target enthält,

   wobei
   die erste und die zweite Probe der Substanz mit unterschiedlichen Stoffmengen des immobilisierten Targets und der gleichen Stoffmenge des gelösten Targets inkubiert werden, die dritte und die vierte Probe der Substanz mit den Stoffmengen des immobilisierten Targets wie die erste und die zweite Probe der Substanz und mit einer gleichen Stoffmenge des gelöstes Targets inkubiert werden,
   wobei sich die Stoffmenge des gelösten Targets in den Probenbehältnissen drei und vier von der Stoffmenge des gelöstes Targets in den Probenbehältnissen eins und zwei unterscheidet und
   die Probenbehältnisse bei der Inkubation das gleiche Volumen an flüssiger Phase, bestehend aus Pufferlösung, gelöstem Target und Substanzprobe, enthalten,
   weiterhin umfassend die Schritte

   • Abtrennen des festen, vorzugsweise partikulären, Trägers aus den jeweiligen Inkubationsansätzen,
   • Messung der Konzentration der nicht an das immobilisierte Target gebundenen Substanz oder des nicht an das immobilisierte Target gebundenen Substanzgemisches (APA-Konzentration) im Überstand des jeweiligen Inkubationsansatzes,
   • Bestimmung einer Bindungskonstante der Substanz oder des Substanzgemisches zum immobilisierten Target und einer Bindungskonstante der Substanz oder des Substanzgemisches zum gelösten Target mit Hilfe der gemessenen APA-Konzentrationen,
   • wobei es sich bei den Bindungskonstanten um Dissoziationskonstanten handelt und die Bestimmung der Dissoziationskonstanten mit Hilfe der Gleichung I

$$APA = \frac{c_0 \cdot K_D^H \cdot ([P] + K_D^P)}{[\text{immoT}] \cdot K_D^P + K_D^H \cdot ([P] + K_D^P)} \ (\text{I})$$

   erfolgt, wobei

      APA die Konzentration der Substanz bezeichnet, die nicht an das immobilisierten Target gebunden ist, d.h. freie Substanz und an gelöstem Target gebundene Substanz,

      $K_D^H$ die Dissoziationskonstante von dem immobilisierten Target,

      $K_D^P$ die Dissoziationskonstante von dem gelösten Target,
      $c_0$ die gesamte konstant zugegebene Substanzkonzentration,

[immoT] die Konzentration des immobilisierten Targets und

[P] die Konzentration des gelösten Targets bezeichnet.

2. Verfahren nach Anspruch 1, **dadurch gekennzeichnet, dass** die Bestimmung der Dissoziationskonstanten weiterhin folgende Schritte umfasst:

• Einsetzen einer Matrix von Dissoziationskonstanten für $K_D^H$ und $K_D^P$ in die Gleichung I,

• Berechnung der jeweiligen zu erwartenden APA Konzentrationen für die Matrix von Dissoziationskonstanten,
• Vergleich der berechneten APA Konzentrationen mit der gemessenen APA Konzentration,

• Auswahl des Wertepaars $K_D^H$ und $K_D^P$, das zur kleinsten Abweichung zwischen berechneter APA Konzentration und gemessener APA Konzentration führt als bestimmte Dissoziationskonstanten der zu untersuchenden Substanz in Bezug auf das immobilisierte bzw. das gelöste Target.

3. Verfahren nach Anspruch 2, **dadurch gekennzeichnet, dass** die Auswahl des Wertepaares $K_D^H$ und $K_D^P$, das zur kleinsten Abweichung zwischen berechneter APA Konzentration und gemessener APA Konzentration führt, durch ein numerisches Optimierungsverfahren erfolgt.

4. Verfahren nach einem der vorhergehenden Ansprüche, **dadurch gekennzeichnet, dass** die Konzentration der Substanz oder des Substanzgemisches (APA) im Überstand des jeweiligen Inkubationsansatzes relativ zu einer Referenzprobe bestimmt wird.

5. Verfahren nach einem der vorhergehenden Ansprüche, **dadurch gekennzeichnet, dass** das gelöste und das immobilisierte Target identisch oder unterschiedlich sind.

6. Verfahren nach einem der vorhergehenden Ansprüche, **dadurch gekennzeichnet, dass** der Träger in wässriger Lösung unlöslich ist.

7. Verfahren nach einem der vorhergehenden Ansprüche, **dadurch gekennzeichnet, dass** der Träger aus organischem oder anorganischem Polymer, insbesondere, Agarose besteht.

8. Verfahren nach einem der vorhergehenden Ansprüche, **dadurch gekennzeichnet dass** der Träger partikulär vorliegt, wobei die Partikel zumindest teilweise mikro- oder nanoskalig sind.

9. Verfahren nach einem der vorhergehenden Ansprüche, **dadurch gekennzeichnet, dass** das immobilisierte Target Albumin ist.

10. Verfahren nach einem der vorhergehenden Ansprüche, **dadurch gekennzeichnet, dass** das lösliche Target Plasma oder Serum von humanem oder tierischem Ursprungs ist.

11. Verfahren nach einem der vorhergehenden Ansprüche, **dadurch gekennzeichnet, dass** das Abtrennen des Trägers durch Separierung des Trägers durch Anlegen eines magnetischen Feldes erfolgt.

12. Verfahren nach einem der vorhergehenden Ansprüche, **dadurch gekennzeichnet, dass** die Messung der Konzentration der Substanz oder des Substanzgemisches durch massenspektrometrische, fluoreszenzspektroskopische, radioaktive oder chromatographische Verfahren oder eine Kombination dieser Verfahren erfolgt.

13. Verfahren nach einem der vorhergehenden Ansprüche **dadurch gekennzeichnet, dass** die Probenbehältnisse Kavitäten von Mikrotiterplatten sind oder Oberflächeneigenschaften aufweisen, die nicht mit der Substanz oder dem Substanzgemisch interferieren

**Claims**

1. Method for determining binding constants of a substance or of a substance mixture with reference to a dissolved target and an immobilised target, comprising the steps

• incubating a first sample of the substance or of the substance mixture with a target immobilised on a solid, preferably particulate, carrier in a first sample container, which contains a buffer solution and a dissolved target,
• incubating a second sample of the substance or of the substance mixture with the target immobilised on a solid, preferably particulate, carrier in a second sample container, which contains buffer solution and dissolved target,
• incubating a third sample of the substance or of the substance mixture with the target immobilised on a solid, preferably particulate, carrier in a third sample container, which contains buffer solution and dissolved target,
• incubating a fourth sample of the substance or of the substance mixture with the target immobilised on a solid, preferably particulate, carrier in a fourth sample container, which contains buffer solution and dissolved target,

wherein
the first and the second sample of the substance are incubated with different material quantities of the immobilised target and the same material quantity of the dissolved target,
the third and fourth sample of the substance are incubated with the material quantities of the immobilised target like the first and second sample of the substance and with an identical material quantity of the dissolved target,
wherein the material quantity of the dissolved target in the sample containers three and four differs from the material quantity of the dissolved target in the sample containers one and two
and
the sample containers in the incubation contain the same volume of liquid phase, consisting of buffer solution, dissolved target and substance sample,
furthermore comprising the steps

• separation of the solid, preferably particulate, carrier out of the respective incubation batches,
• measurement of the concentration of the substance which is not bound to the immobilised target or of the substance mixture which is not bound to the immobilised target (APA-concentration) in the supernatant of the respective incubation batch,
• determination of a binding constant of the substance or of the substance mixture to the immobilised target and of a binding constant of the substance or of the substance mixture to the dissolved target with the help of the measured APA-concentrations,
• wherein the binding constants are dissociation constants and the determination of the dissociation constants is achieved with the help of the equation I

$$APA = \frac{c_0 \cdot K_D^H \cdot ([P] + K_D^P)}{[\text{immoT}] \cdot K_D^P + K_D^H \cdot ([P] + K_D^P)} \quad (I)$$

wherein

APA denotes the concentration of the substance which is not bound to the immobilised target, i.e. free substance and substance bound to dissolved target,
$K_D^H$ denotes the dissociation constant from the immobilised target,
$K_D^P$ the dissociation constant from the dissolved target,
$c_0$ the total constantly added substance concentration,
[immoT] the concentration of the immobilised target and
[P] the concentration of the dissolved target.

2. Method according to claim 1, **characterised in that** the determination of the dissociation constants furthermore comprises following steps:

• insertion of a matrix of dissociation constants for $K_D^H$ and $K_D^P$ into the equation I,
• calculation of the respective expected APA concentrations for the matrix of dissociation constants,
• comparison of the calculated APA concentrations with the measured APA concentration,
• selection of the value pair $K_D^H$ and $K_D^P$, which leads to the smallest divergence between calculated APA concentration and measured APA concentration as specified dissociation constants of the substance to be tested with respect to the immobilised or the dissolved target.

3. Method according to claim 2, **characterised in that** the selection of the value pair $K_D^H$ and $K_D^P$, which leads to the smallest divergence between calculated APA concentration and measured APA concentration is achieved by a numerical optimisation method.

4. Method according to any of the preceding claims, **characterised in that** the concentration of the substance or of the substance mixture (APA) is determined in the supernatant of the respective incubation batch relatively to a reference sample.

5. Method according to any of the preceding claims, **characterised in that** the dissolved and the immobilised target are identical or different.

6. Method according to any of the preceding claims, **characterised in that** the carrier is insoluble in aqueous solution.

7. Method according to any of the preceding claims, **characterised in that** the carrier consists of organic or inorganic polymer, in particular agarose.

8. Method according to any of the preceding claims, **characterised in that** the carrier is present in particulate form, wherein the particles are at least in part microscale or nanoscale.

9. Method according to any of the preceding claims, **characterised in that** the immobilised target is albumin.

10. Method according to any of the preceding claims, **characterised in that** the soluble target is plasma or serum of human or animal origin.

11. Method according to any of the preceding claims, **characterised in that** the separating of the carrier is achieved by separating the carrier through the application of a magnetic field.

12. Method according to any of the preceding claims, **characterised in that** the measurement of the concentration of the substance or of the substance mixture is achieved by mass spectrometric, fluorescence spectroscopic, radioactive or chromatographic methods or a combination of these methods.

13. Method according to any of the preceding claims **characterised in that** the sample containers are cavities of microtitre plates or have surface characteristics which do not interfere with the substance or with the substance mixture

**Revendications**

1. Procédé de détermination de constantes de liaison d'une substance ou d'un mélange de substances par rapport à une cible dissoute et une cible immobilisée, comprenant les étapes de

• incubation d'un premier échantillon de la substance ou du mélange de substances avec une cible immobilisée sur un support solide, de préférence particulaire, dans un premier récipient d'échantillon, lequel contient une solution tampon et une cible dissoute,
• incubation d'un deuxième échantillon de la substance ou du mélange de substances avec la cible immobilisée sur un support solide, de préférence particulaire, dans un deuxième récipient d'échantillon, lequel contient de la solution tampon et de la cible dissoute,
• incubation d'un troisième échantillon de la substance ou du mélange de substances avec la cible immobilisée sur un support solide, de préférence particulaire, dans un troisième récipient d'échantillon, lequel contient de la solution tampon et de la cible dissoute,
• incubation d'un quatrième échantillon de la substance ou du mélange de substances avec la cible immobilisée sur un support solide, de préférence particulaire, dans un quatrième récipient d'échantillon, lequel contient de la solution tampon et de la cible dissoute,

dans lequel
le premier et le deuxième échantillon de la substance sont incubés avec différentes quantités de matière de la cible immobilisée et la même quantité de matière de la cible dissoute,
le troisième et le quatrième échantillon de la substance sont incubés avec les quantités de matière de la cible immobilisée comme le premier et le deuxième échantillon de la substance et avec une même quantité de matière de la cible dissoute,
dans lequel la quantité de matière de la cible dissoute dans les récipients d'échantillon trois et quatre se distingue de la quantité de matière de la cible dissoute dans les récipients d'échantillon un et deux
et
les récipients d'échantillon contiennent lors de l'incubation le même volume de phase liquide, constituée par une solution tampon, une cible dissoute et un échantillon de substance,
comprenant en outre les étapes de

• séparation du support solide, de préférence particulaire, des préparations d'incubation respectives,
• mesure de la concentration de la substance non liée à la cible immobilisée ou du mélange de substances non lié à la cible immobilisée (concentration APA) dans le surnageant de la préparation d'incubation respective,
• détermination d'une constante de liaison de la substance ou du mélange de substances pour la cible immobilisée et d'une constante de liaison de la substance ou du mélange de substances pour la cible dissoute à l'aide des concentrations APA mesurées,
• dans lequel les constantes de liaison sont des constantes de dissociation et la détermination des constantes de dissociation se fait à l'aide de l'équation I

$$APA = \frac{c_0 \cdot K_D^H \cdot ([P] + K_D^P)}{[\text{immoT}] \cdot K_D^P + K_D^H \cdot ([P] + K_D^P)} \quad (I)$$

dans lequel

APA désigne la concentration de la substance, qui n'est pas liée à la cible immobilisée, autrement dit substance libre et substance liée à la cible dissoute,

$K_D^H$ la constante de dissociation de la cible immobilisée,

$K_D^P$ la constante de dissociation de la cible dissoute,

$c_0$ la concentration de substance totale ajoutée de manière constante,

[immoT] la concentration de la cible immobilisée et

[P] la concentration de la cible dissoute.

2. Procédé selon la revendication 1, **caractérisé en ce que** la détermination des constantes de dissociation comprend en outre les étapes suivantes :

• utilisation d'une matrice de constantes de dissociation pour $K_D^H$ et $K_D^P$ dans l'équation I,
• calcul des concentrations APA attendues respectives pour la matrice de constantes de dissociation,
• comparaison des concentrations APA calculées avec la concentration APA mesurée,
• sélection du couple de valeurs $K_D^H$ et $K_D^P$, qui entraîne le plus petit écart entre la concentration APA calculée et la concentration APA mesurée en tant que constantes de dissociation déterminées de la substance à analyser par rapport à la cible immobilisée ou à la cible dissoute.

3. Procédé selon la revendication 2, **caractérisé en ce que** la sélection du couple de valeurs $K_D^H$ et $K_D^P$, qui entraîne le plus petit écart entre la concentration APA calculée et la concentration APA mesurée, se fait par un procédé d'optimisation numérique.

4. Procédé selon l'une quelconque des revendications précédentes, **caractérisé en ce que** la concentration de la substance ou du mélange de substances (APA) dans le surnageant de la préparation d'incubation respective est déterminée par rapport à un échantillon de référence.

5. Procédé selon l'une quelconque des revendications précédentes, **caractérisé en ce que** la cible dissoute et la cible immobilisée sont identiques ou différentes.

6. Procédé selon l'une quelconque des revendications précédentes, **caractérisé en ce que** le support est insoluble dans une solution aqueuse.

7. Procédé selon l'une quelconque des revendications précédentes, **caractérisé en ce que** le support est constitué de polymère organique ou anorganique, en particulier, d'aga rose.

**8.** Procédé selon l'une quelconque des revendications précédentes, **caractérisé en ce que** le support est disponible sous forme particulaire, dans lequel les particules sont au moins en partie à l'échelle micro ou nano.

**9.** Procédé selon l'une quelconque des revendications précédentes, **caractérisé en ce que** la cible immobilisée est de l'albumine.

**10.** Procédé selon l'une quelconque des revendications précédentes, **caractérisé en ce que** la cible soluble est du plasma ou sérum d'origine humaine ou animale.

**11.** Procédé selon l'une quelconque des revendications précédentes, **caractérisé en ce que** la séparation du support se fait par séparation du support par application d'un champ magnétique.

**12.** Procédé selon l'une quelconque des revendications précédentes, **caractérisé en ce que** la mesure de la concentration de la substance ou du mélange de substances se fait par procédé de spectrométrie de masse, de spectroscopie de fluorescence, radioactif ou chromatographique ou une combinaison de ces procédés.

**13.** Procédé selon l'une quelconque des revendications précédentes, **caractérisé en ce que** les récipients d'échantillon sont des cavités de plaques microtitres ou présentent des propriétés de surface, qui n'interfèrent pas avec la substance ou le mélange de substances.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102010018965 A1
- EP 1658499 A1

- WO 2005017528 A